Europäisches Patentamt

European Patent Office

Office européen des brevets

(19)

(11) Publication number: **0 253 661**
A2

# (12) EUROPEAN PATENT APPLICATION

(21) Application number: 87306293.9

(51) Int. Cl.⁴: **B 01 D 9/00**

(22) Date of filing: 16.07.87

(30) Priority: 17.07.86 AU 7003/86

(43) Date of publication of application:
20.01.88 Bulletin 88/03

(84) Designated Contracting States:
AT BE CH DE ES FR GB GR IT LI LU NL SE

(71) Applicant: BRACTON CHEMICAL CO. PTY. LTD.
50 Chard Road
Brookvale New South Wales 2100 (AU)

(72) Inventor: Hunter, Michael Jeffrey
50 Chard road
Brookvale, New South Wales (AU)

(74) Representative: Silverman, Warren et al
HASELTINE LAKE & CO. Hazlitt House 28 Southampton
Buildings Chancery Lane
London WC2A 1AT (GB)

(54) Method of concentrating crystalline product.

(57) A crystalline component of a crystalline composition is obtained in a concentrated state by raising the temperature of said composition to cause melting thereof enabling impurities of a different density to said component to be drained; allowing said composition to recrystallize; and placing the recrystallized composition under pressure in a press to remove therefrom liquid impurities entrapped with the crystals of said component. This procedure is particularly applicable to the concentration of p-dichlorobenzene.

EP 0 253 661 A2

Bundesdruckerei Berlin

# Description

## METHOD OF CONCENTRATING CRYSTALLINE PRODUCT

The present invention relates to a method of concentrating a crystalline substance having a melting point below the temperature at which it will decompose. More particularly, but not exclusively, the present invention relates to a method of concentrating para-dichlorobenzene.

Current methods and apparatus used to concentrate p-dichlorobenzene are generally expensive due to the large capital investment required. The price of p-dichlorobenzene is accordingly increased as a result thereof.

It is an object of the present invention, to overcome or substantially ameliorate the above disadvantage.

There is disclosed herein a method of concentrating a crystalline component of a crystalline composition; said method including the steps of raising the temperature of said composition to cause melting thereof enabling impurities of a different density to said component to be drained; allowing said composition to recrystallize; and placing the recrystallized composition under pressure in a press to remove therefrom liquid impurities entrapped with the crystals of said component.

In the preferred form of the present invention the above-mentioned crystalline component is p-dichlorobenzene and said crystalline composition includes other dichlorobenzenes such as ortho-dichlorobenze and meta-dichlorobenzene. There may also be present in the composition chlorobenzene, chlorotoluenes and chlorothiophenes and water. The composition is raised to a temperature at or above 53°C, but preferably about 80°C. When the crystalline composition has melted, the major part of the water can be removed simply by density separation. More particularly, the water will float allowing it to be drained. When the composition is recrystallized the o- and m-dichloro-benzenes as well as the chlorobenzene, chlorotoluenes and chlorothiophenes are still in a liquid state enabling them to be drained together with any water should it still be present if it has not previously been drained. The remaining recrystallized composition is then placed in a press and subjected to pressure. Further o- and m-dichlorobenzene is extracted together with chlorobenzene, chlorotoluenes and chlorothiophenes.

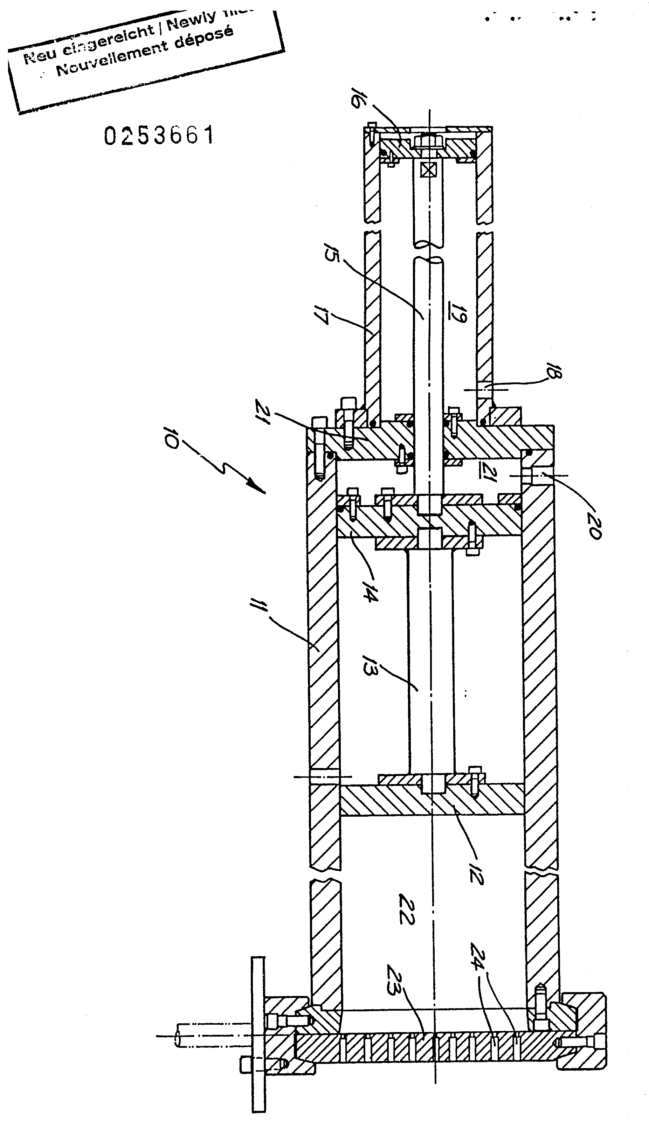

A preferred form of the present invention will now be described by way of example with reference to the accompanying drawing which schematically depicts in sectioned side elevation a press to be used in the concentration of p-dichlorobenzene.

When para-dichlorobenzene is purchased commercially, the composition actually purchased usually only consists of about 81% p-dichlorobenzene. The remainder is made up of water, usually about 6%, together with o- and m-dichlorobenzene, chlorobenzene, chlorotoluenes and chlorothiophenes.

When the above is melted the water will separate from the dichlorobenzenes as it has a different density. The water can be separated at this time or, alternatively, the composition can be allowed to recrystallize and then the water drained. Once the composition has been recrystallized, the water at this stage can be drained together with any other liquid impurities. The recrystallized composition is then placed in a press as depicted in the attached drawing.

The press 10 includes a cylinder wall 11 which cooperates with a piston 12. Extending from the piston 12 is a push rod 13 connected to a further piston 14. Extending from the piston 14 is a push rod 15 attached to a still further piston 16. The piston 16 is sealingly located within a further cylinder 17. The cylinder 17 is provided with a port 18 allowing liquid to enter and leave the cavity 19 defined by the cylinder 17 and the piston 16. The cylinder 11 is provided with a port 20 allowing liquid to be delivered and removed from the chamber 21 defined by the cylinder 11, piston 14 and end wall 21. In operation of the press 10, the recrystallized composition is placed in the chamber 22 and placed under pressure by delivering hydraulic fluid under pressure to the chamber 21. This causes movement of the piston 14 and therefore movement of the piston 12. The chamber 22 is closed by means of a plate 23 provided with a plurality of passages 24. As the composition within the chamber 22 is subjected to pressure, liquid contaminants are forced through the passages 24. As the pressure continues to rise, the crystals are then forced through the passages 24. The piston 12 is moved back to the start position by delivering hydraulic fluid under pressure to the chamber 19. Port 20 then acts as a drain in respect of the chamber 21.

With the piston 12 of the above-described press 10 having a diameter of approximately 300 mm, a pressure of approximately 6.9 to 8.275 MPa (1000 to 1200 psi) is applied to the piston 12 in order to force the liquid contaminants through the passages 24. However, it should be appreciated that the majority of the crystals are removed through the passages 24 at higher pressures, i.e. about 16.5 MPa (2400 psi).

With these pressures the passages 24 are about 12 mm in diameter. Preferably the passages 24 are 12 mm in diameter for approximately 12 mm, and then increased in size to about 20 mm. The smaller ends communicating with the chamber 22.

By using the above described method and apparatus, p-dichlorobenzene can be manufactured with a purity of 99.5%. In a typical sample tested, the p-dichlorobenzene manufactured did not contain any chlorobenzenes. The liquid collected from the press 10 contained o- and m-dichlorobenzenes, chlorobenzene, chlorotoluenes and chlorothiophenes. There was also present some p-dichlorobenzene. Typically, the liquid collected would consist of approximately 46.5% p-dichlorobenzene, 34.8% o-dichlorobenzene, 4.6% m-dichlorobenzene, 10% chlorobenzene, 1.8% chlorotoluenes, and 1.9% chlorothiophenes.

## Claims

1. A method of concentrating a crystalline component of a crystalline composition; said method including the steps of raising the temperature of said composition to cause melting thereof enabling impurities of a different density to said component to be drained; allowing said composition to recrystallize; and placing the recrystallized composition under pressure in a press to remove therefrom liquid impurities entrapped with the crystals of said component.

2. The method of claim 1, wherein said crystalline component is p-dichlorobenzene.

3. The method of claim 2, wherein said composition is raised to a temperature at or above 53°C.

4. The method of claim 3, wherein said composition is raised to a temperature of about 80°C.

5. The method of any one of claims 2 to 4, wherein said crystalline composition includes chlorobenzene, chlorotoluenes, chlorothiophenes and water.

6. The method of any preceding claim, wherein said impurities are removed in said press by being forced through passages extending through an end wall of said press.

7. The method of claim 6, wherein said crystalline composition is first subjected to a pressure of from 6.9 to 8.275 MPa (1000 to 1200 psi) and said passages have a diameter of approximately 12 mm.

8. The method of claim 7, wherein said crystalline composition is further subjected to a pressure of about 16.5 MPa (2400 psi).

0253661